(19) Europäisches Patentamt
European Patent Office
Office européen des brevets

(11) **EP 4 403 205 A2**

(12) **EUROPEAN PATENT APPLICATION**

(43) Date of publication:
**24.07.2024 Bulletin 2024/30**

(21) Application number: **24164654.6**

(22) Date of filing: **09.12.2020**

(51) International Patent Classification (IPC):
***A61N 1/04*** *(2006.01)*

(52) Cooperative Patent Classification (CPC):
**A61N 1/0492**; Y02E 60/36

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.12.2019 US 201962946124 P**

(62) Document number(s) of the earlier application(s) in
accordance with Art. 76 EPC:
**20838717.5 / 4 072 652**

(71) Applicant: **Candesant Biomedical, Inc.
San Francisco, CA 94118 (US)**

(72) Inventors:
• **HUNT, Niquette
San Francisco, 94118 (US)**

• **WAUGH, Jacob M.
San Francisco, 94118 (US)**
• **ROSEN, Jesse
San Francisco, 94118 (US)**
• **O'NEIL, Michael
San Francisco, 94118 (US)**

(74) Representative: **Sagittarius IP
Marlow International
Parkway
Marlow SL7 1YL (GB)**

Remarks:
•This application was filed on 19-03-2024 as a
divisional application to the application mentioned
under INID code 62.
•Claims filed after the date of receipt of the divisional
application (Rule 68(4) EPC).

(54) **METHODS AND SYSTEMS FOR DISPOSING ALKALI METAL PATCHES**

(57) A method for disposing of a device including an alkali metal is described. In one embodiment, the method includes placing the device in a container configured to permit a controlled exposure of the alkali metal to a reactant for the alkali metal or a solubilizer of the alkali metal, and allowing the alkali metal to react with the reactant or to dissolve in the solubilizer to render the alkali metal substantially non-reactive. Containers for use in the method and kits including the alkali metal device and the disposal container are also described.

FIG. 2

**Description**

CROSS-REFERENCE TO RELATED APPLICATIONS

The application claims the benefit of U.S. Provisional Patent Application No. 62/946,124, filed December 10, 2019, which is hereby incorporated by reference in its entirety.

TECHNICAL FIELD

The technology described herein generally relates to methods and devices to dispose alkali metal patches after use by a patient under a therapeutic procedure.

BACKGROUND

Disposable medical devices for use by patients may include amounts of highly reactive materials, compounds, or elements. The disposal of such devices after use poses a hazardous challenge for the patients, for the medical personnel, and for the facility storing the disposal materials. In some configurations, it may be desirable to neutralize the disposable medical device completely, prior to, or in conjunction with, disposing of it. In the case of medical devices containing alkali materials, effective disposal methodologies that can be applied with simplicity, rapidly, and securely are lacking or non-existent.

SUMMARY

In a first embodiment, a method for disposing of a device having an alkali metal includes placing into a container a device including a layer portion having at least an alkali metal, an oxide of the alkali metal, a hydroxide of the alkali metal, or any combination thereof. The method also includes controllably exposing the layer portion on the device to a reactant for the alkali metal or a solubilizer of the alkali metal and allowing the alkali metal to react with the reactant or to dissolve in the solubilizer to render the alkali metal substantially non-reactive. The method also includes optionally disposing of the device, the container, or both.

In a second embodiment, a kit is disclosed that includes a device including a layer of an alkali metal and a disposal container. The disposal container has a cavity configured to receive the device and an opening configured to receive the device into the cavity, the opening closable or capable of being closed. The disposal container also includes one or more of: (i) a solvent that dissolves the alkali metal, (ii) a means for the egress of hydrogen or a scavenger of hydrogen, and (iii) a source of reactant or a mechanism to receive a reactant from an external source. In one embodiment, the reactant is water.

In a third embodiment, a method is disclosed that includes sealing, in a disposal container, a device including a layer of an alkali metal and an oxide or a hydroxide of the alkali metal. The disposal container includes a closeable opening and a semi-permeable membrane. The method also includes allowing a water vapor molecule to contact the layer and controllably oxidizing the alkali metal to form an alkali metal oxide or hydroxide and to generate a hydrogen molecule. The method also includes allowing the hydrogen molecule to egress the disposal container.

In yet another embodiment, a container includes an enclosure forming a cavity and configured to receive a device including an alkali metal layer in the cavity and a medium configured to allow contact of a water molecule or a reactant with the alkali metal at a pre-selected rate or in a controlled manner.

BRIEF DESCRIPTION OF THE DRAWINGS

FIG. 1 illustrates a kit for disposing of a used patch, according to some embodiments.
FIG. 2 illustrates a kit for disposing of a used patch that is put in contact with a coated mesh, according to some embodiments.
FIG. 3 illustrates a kit for disposing of a used patch including a first compartment and a second compartment, according to some embodiments.
FIG. 4 illustrates a kit for disposing of a used patch including a first compartment, a second compartment, and a third compartment, according to some embodiments.
FIG. 5 illustrates a container for disposing of a used patch including a cylindrical compartment, according to some embodiments.
FIG. 6 illustrates a container for disposing of multiple used patches, according to some embodiments.

FIG. 7 illustrates a device for disposing of a used patch, according to some embodiments.
FIG. 8 is a flow chart illustrating steps in a method for disposing of a used patch, according to some embodiments.
FIG. 9 is a flow chart illustrating steps in a method for disposing of a used patch, according to some embodiments.

## DETAILED DESCRIPTION

Devices including an alkali metal (*e.g.*, lithium, sodium, potassium, rubidium, cesium, francium, or any combination of the above) enable an approach for effective treatment of certain skin conditions, such as hyperhidrosis (the profusion of sweat) or other medical or aesthetic dermatological conditions. The alkali metal devices can be applied to a human body surface, such as skin, for such medical therapy. In some embodiments, the alkali metal device may include a pen-like device, or stylus for wound closure. The device has a core made of a thin, elongated column of alkali metal (*e.g.*, a mechanical pencil with a ¼ inch diameter led made from the alkali metal). The user applies the pen over a wound, tracing a thin layer of alkali metal over the tissue, for healing. The slow reaction of the alkali metal with the skin moisture and ambient air creates a gentle amount of heat that promotes tissue recovery.

The devices can also be used to substantially sterilize or render substantially aseptic a surface - a human skin surface or any other surface, such as a surgical instrument, a laboratory bench top surface, or a petri dish. Approaches for safe and easy disposal of the devices are needed, whether the device is used in a controlled environment of a healthcare facility (*e.g.*, hospital, clinic, doctor's office), in a laboratory, or in a home setting.

Alkali metals (*e.g.*, sodium, potassium lithium, rubidium, cesium, or francium) are highly reactive. The reaction is typically vigorous and exothermic, sometimes melting the metal, sometimes igniting the evolved hydrogen, and sometimes inducing a Coulomb explosion. Embodiments as disclosed herein include methods and systems to safely neutralize and dispose of alkali metal-based skin patches, pens, and other related therapeutic devices by the end user (*e.g.*, in a hospital, doctor office, clinic, or eventually patient household settings). Methods and devices disclosed herein allow the alkali metal or alkali metal oxide or hydroxide to chemically react in a controlled manner, avoiding the excess temperatures or reaction conditions that may melt the alkali metal or ignite evolved hydrogen ($H_2$) or related flammable gasses or materials. Some embodiments include commercial/residential waste streams, *e.g.*, city drainage or trash disposal means.

Accordingly, disclosed herein are methods and devices to neutralize, render harmless, deactivate, and/or consume an alkali metal in a device including an alkali metal layer. When the alkali metal reacts with water, *e.g.*, during use of the device when the alkali metal layer contacts sweat or another source of water, energy (*e.g.*, heat) is generated from the exothermic reaction between water and the alkali metal. In some embodiments, the alkali metal may include an alloy of an alkali metal, or any compound having less than 100% alkali metal in it. The energy is transferred to the treatment surface, such as a skin or body surface of a human, to provide a clinical benefit. In some embodiments, the device, also referred to herein as a patch, is configured for a single-use and to be disposable, and the single-use may leave a portion of the alkali metal unreacted - e.g., a portion of the alkali metal remains capable of reaction with water. Accordingly, it is desirable to have a simple, safe way to react the unused or unreacted portion of alkali metal, to render it safe so that it may be disposed of with conventional methods (*e.g.*, mixed into a solution that can be drained or placed in a garbage container).

A reaction of an alkali metal compound (*e.g.*, sodium, Na; potassium, K) with water (*e.g.*, the "reactant" or "solubilizer") may be described with the following chemical equation:

$$\underbrace{Na}_{Alkali\ Metal} + \underbrace{H_2O}_{Reactant} \rightarrow \underbrace{Na^+_{(aq)} + OH^-_{(aq)} + \tfrac{1}{2} \cdot H_{2(g)} + energy}_{Product} \qquad (1)$$

The reaction in Eq. 1, where Na is used as an example for illustrative purposes only, is highly exothermic, and may in fact produce a harmful, explosive shock. To avoid the explosive shock, it is important to limit the rate of the reaction. Moreover, a sudden rise in temperature from the heat may further lead to ignition of the hydrogen, $H_2$, especially when a sizeable amount of hydrogen has accumulated after a certain amount of time. For instance, when sodium metal contacts liquid water, a large quantity of heat is generated along with hydrogen. The heat may cause the alkali metal to melt, generating liquid metallic droplets that may detach from the alkali metal that composes the device, further worsening the hazard. Methods and devices as disclosed herein avoid the above undesirable effects, providing a user-friendly disposal means.

Another challenge is the removal of the residual hydrogen, steam, and related gasses. For example, in some embodiments, a used patch may include an alkali layer with dimensions 100 mm × 100 mm × 0.1 mm (1000 mm$^3$ = 1 mL = 1 cm$^3$ of Na). With a density of $\rho_{Na}$ = 0.97g/cm$^3$, and a molar weight (MW = 23g/mol), this results in 0.97/23 = 0.0422 mol of Na in the used patch. According to the stoichiometry of Eq. 1, this corresponds to: 0.0422/2 mol (of $H_2$) ÷ 22.4 L/mol (molar volume of $H_2$ at STP) = 473 mL $H_2$ per Patch. That is, in some embodiments, a single-used patch neutralization produces almost half a liter of highly flammable hydrogen gas. Accordingly, embodiments such as those disclosed herein may include: hydrogen scavenging materials, solutions, and/or structures, purging inert gases, valves, and membranes to trap or allow the egress of hydrogen and related gasses that are generated during the reaction.

Embodiments as disclosed herein provide various solutions to convert the alkali metal residue in a used patch into another, non-pyrophoric substance, such as a metal hydroxide, a metal alkoxide, or a salt (*e.g.*, an alkali-halide such as NaCl, and the like).

Accordingly, embodiments include a disposal means having containers with one or more compartments and are configured to limit the chemical reaction (*cf.* Eq. 1) so as to avoid any possible explosive conditions. Disposal kits as disclosed herein control the rate of the reaction to neutralize the alkali metal, and completely consume the residual alkali metal in the patch to yield a non-pyrophoric substance that can be discarded in standard waste streams (*e.g.*, garbage containers, drains, or similar means). In some embodiments, a disposal kit may contain and manage the heat generated by the reaction such that it is safe for the user. For example, in some embodiments, the exterior portion of a container as disclosed herein is safely maintained below 50 °C.

Disposal kits as disclosed herein may include vents, absorbing materials, gels, solutions, or other controls to manage products including gas(es), *e.g.*, hydrogen, that are involved in the reaction described in Eq. 1 or equivalents.

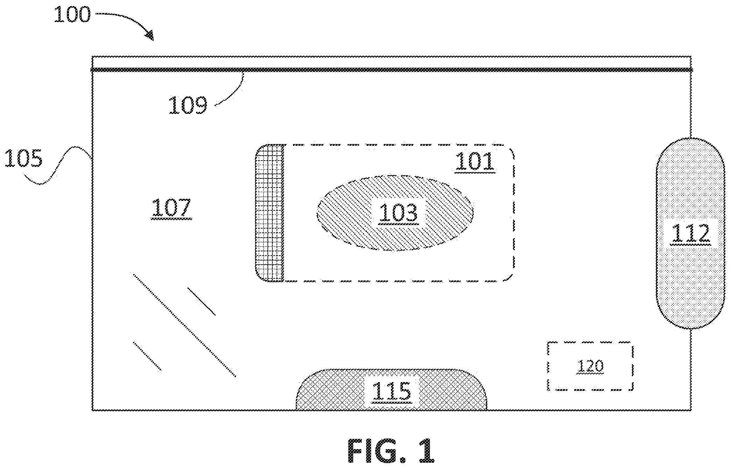

FIG. 1 illustrates a disposal kit 100 for disposing of a used patch 101, according to some embodiments. Used patch 101 is generically a device including a layer of an alkali metal 103. In some embodiments, disposal kit 100 may be configured as a single-use disposal unit (*e.g.*, the user disposes of at least a portion of disposal kit 100 after used patch 101 is neutralized). In some embodiments, disposal kit 100 may be configured to receive and neutralize multiple used patches 101 before being discarded. In yet other embodiments, disposal kit 100 may be configured to receive and neutralize multiple used patches 101 that are removed from disposal kit 100 when, for example, an indicator means signals that it is safe to do so. In such embodiments, the used, neutralized alkali patches 101 may be removed from disposal kit 100 and safely discarded to allow the continued introduction of further used patches 101.

Disposal kit 100 includes a disposal container 105 including a cavity 107 and an opening 109 configured to receive used patch 101 into cavity 107. In some embodiments, container 105 includes a packaging that is substantially impermeable to water and to air, having a closeable opening 109. In some embodiments, closeable opening 109 includes a sliding lock (*e.g.*, a Ziploc top) or screw cap to seal the used patch in the disposal container. In some embodiments, disposal container 105 is manufactured at least in part from a water-permeable material. In some embodiments, disposal container 105 may include a polyethylene or polypropylene vial or a poly bag. Further, in some embodiments, disposal container 105 may include at least one or more walls (*e.g.*, a front wall and a rear wall), where the bottom wall is attached to the front and rear walls. In some embodiments, disposal container 105 further includes two side walls, where each side wall is connected to the front wall, the rear wall, and to the bottom wall, wherein at least one wall is flexible. In some embodiments, at least one of the walls is circular or curved, and disposal container 105 includes an annular or a circular cavity. In some embodiments, at least one of the walls in the container may include a metal, a metal foil (*e.g.*, stainless steel, or Mylar), or a metal-sputtered plastic film.

In one embodiment, disposal container 105 may have a venting cap or valve 112 to allow a hydrogen (or any other excess gas) egress from and allows water into, disposal container 105.

In some embodiments, disposal container 105 includes a reactant 115 that chemically interacts with alkali metal 103 in used patch 101 to neutralize it. Reactant 115 may include a solvent that is miscible with water and that dissolves alkali metal 103. The solvent may include an alcohol or a glycol, wherein the alcohol is selected from ethanol, isopropanol, t-butanol, stearyl alcohol, and tris(trimethylsilyl)methanol, and the glycol includes propylene glycol. In some embodiments, the reactant may include a few milliliters of substantially anhydrous R-OH (~99%) solution, where R is an unspecified chemical group. For example, in some embodiments, reactant 115 may include ethanol, isopropanol, t-butanol, or heavier, unusual or sterically hindered alcohols having a more predictable and slow reactivity. In some embodiments, reactant 115 may include a low viscosity or gelled solution to react with alkali metal 103. In some embodiments, the water or water solution is contained on or in a sponge, a porous body, or an absorbent polymer substrate. In some embodiments, the absorbent polymer substrate may be a hydrogel including a cross-linked hydrophilic polymer. In some embodiments, reactant 115 may include a triglyceride, an anhydrous foam, or a compound with a counter ion that produces upon reacting with sodium or related metals or alloys a compound selected from sodium alginate, sodium difluoride, sodium fluorosilicate, sodium metaborate, sodium paraperiodate, sodium stearate, sodium zirconium glycolate, and sodium perrhenate ($NaReO_4$) in anhydrous ethanol. The addition of sodium or other alkali metals will produce nonahydridorhenate. These are well-behaved reactions that yield inert salts. In some embodiments, the rhenium (Rh) compound may be replaced with more affordable substances such as with technetium (Tc) or manganese (Mn).

Further, in some embodiments, disposal kit **100** may include a mechanism to receive water from an external source. In some embodiments, the reactant may be a compound with a counter ion that produces upon reacting with sodium a compound selected from sodium alginate ($NaC_6H_7O_6$), potassium difluoride ($KHF_2$), sodium difluoride ($NaHF_2$), sodium fluorosilicate ($Na_2SiF_6$), sodium metaborate ($NaBO_2$), sodium paraperiodate ($Na_3H_2IO_6$), sodium stearate ($NaOOCC_{17}H_{35}$), and sodium zirconium glycolate ($NaZrH_3(H_2COCOO)_3$).

In some embodiments, the mechanism to receive water from an external source is a water or moisture-permeable polymer membrane. In addition to water or a solution of water and salt, solutions that may be used to limit the rate of the chemical reaction (Eq. 1) may include: propylene glycol (PG), alcohol, or high molar NaOH(aq) such as 10M NaOH, which slowly reacts with the sodium in a controlled manner.

Additionally, in some embodiments, reactant 115 may include carbon dioxide to form a carbonate of the alkali metal. The $CO_2$ may be in gaseous form or at least one compartment could be filled with a substance that releases carbon dioxide. Additionally, carbon tetrachloride and dichloromethane react vigorously with sodium and could be used as reactant 115 to expend the used sodium.

In some embodiments, it may be beneficial to neutralize the products following the alkali metal reactions (*cf.* Eq. 1). Highly basic alkali hydroxides (*e.g.*, NaOH, *cf.* Eq. 1) are caustic and/or corrosive, and may be a hazardous challenge to dispose of. Accordingly, some embodiments may include buffers, acids, or similar compounds in a solution of reactant 115, to neutralize or control the reaction products (*e.g.*, right hand side of Eq. 1).

One or more walls of cavity 107 may include a membrane that is selectively permeable to hydrogen. Such semi-permeable membrane can be polymeric membranes, porous membranes, dense metal membranes, or ion-conductive membranes. Exemplary porous membranes include ceramic, carbon, and metallic membranes. Exemplary polymer membranes include: aromatic polyimides, polysulfone, cellulose acetate, polyethylene, and tetrabromopolycarbonate. Exemplary dense metal membranes include palladium and palladium-based alloy membranes. The hydrogen permeable membrane can also be a hybrid membrane of nanoparticles dispersed in a polymer matrix, such as those described, for example, in Pulyalina A., et al., Polymers, 10(8): 828 (2018). Some embodiments allow hydrogen to escape while retaining water vapor or steam. In some embodiments, the alkali patch may be partially or totally immersed in the reactant (*e.g.*, a PG solution). Some embodiments control the environment of disposal container 105 so as to provide a high humidity level. For the latter, disposal container 105 may include a membrane that vents hydrogen at a higher rate than water vapor or steam that may be desirable.

In one embodiment, water vapor enters cavity 107 of a pouch and the reaction with alkali metal 103 proceeds (*cf.* Eq. 1). Hydrogen exits cavity 107 through the membrane or material that is hydrogen-permeable, and the alkali metal then oxidizes, leaving behind a high molar hydroxide hydrate, a crust, or layer of alkali hydroxide (*e.g.*, NaOH *cf.* Eq. 1) on the used patch, which is safely sealed in the first cavity. In another embodiment, the hydrogen permeable membrane has a permeation or diffusion rate for hydrogen that is at least about 10%, 20%, 25%, 30%, 35%, 40%, 50%, 60%, 75%, or higher than the permeation rate for water vapor or steam.

In some embodiments, at least the first cavity in the container may include a wall wherein at least a portion of the wall includes a hydrogen-permeable membrane or material (*e.g.*, a synthetic polymer such as those mentioned above, and similar). In some embodiments, the semi-permeable membrane may also be permeable to water vapor or steam. The semi-permeable membrane may be disposed on a bottom wall of disposal container 105. The hydrogen released by the interaction between the reactant and the alkali metal may then egress freely from disposal container 105 through the hydrogen-permeable membrane and be released into the atmosphere. Disposal container 105 including used patch 101 may then be safely discarded using regular procedures. In some embodiments, disposal container 105 also includes a source of reactant (e.g., water or other solvent) or a mechanism to receive a reactant from an external source (*e.g.*, the reaction products on the left-hand side of Eq. 1). In one embodiment, used patch 101 is exposed to water or an aqueous solution in a manner that limits the availability of water for reaction with alkali metal 103 or alloy thereof, thus avoiding an explosive regime. In some embodiments, the reactant may include a membrane with a preselected diffusion gradient that slowly passes water (in liquid or vapor form) across to cavity 107 that includes used patch 101. In some embodiments, reactant 115 includes an aqueous solution, such as a propylene glycol (PG) + water, salt + water, or alcohol + water. In some embodiments, the reactant 115 includes a sponge that entrains water and slowly allows it to ingress into cavity 107 at a limited rate to provide for a controlled reaction.

In some embodiments, reactant 115 may include water or a water solution embedded in a sintered metal, a porous polymer, a porous plastic, or an absorbent polymer substrate (*e.g.*, a hydrogel including a cross-linked hydrophilic synthetic polymer). More specifically, the sintered metal forms a rate-limiting interface between reactant 115 and alkali metal 103 in used patch 101. Further, because the metal has a relatively high thermal mass, the sintered metal could control the temperature of the reacting alkali metal 103 below its melting point and below the auto-ignition temperature of hydrogen. The sintered metal or plastic material or combinations thereof may provide a torturous/porous path to limit the rate of water ingress into cavity 107 that contains used patch 101. In some embodiments, reactant 115 may include beads, a film or sheet of any combination of hydrogel, absorbent or super-absorbent polymer, polyvinyl alcohol (PVA), silicone, or any suitable substrate that contains water or an aqueous solution.

In some embodiments, reactant 115 may include a solution of propylene glycol (PG). Propylene glycol has the benefit of being miscible with water. It is also non-flammable, and safe to use. In some embodiments, the reactant may include a 100% PG solution, or any other ratio of $PG/H_2O$ so as to effectively dissolve the patch. For embodiments in which used patch 101 has an alkali metal 103 with a thin foil sheet (*e.g.*, about ~ .005in of metal or alloy, or less), it may take approximately 30 minutes to completely dissolve the alkali when immersed in a 100% PG solution. Dissolution of alkali metal 103 in used patch 101 occurs faster when the amount of water in the aqueous PG solution increases. In some embodiments, it may be desirable to maintain the level of water in the aqueous PG solution to less than approximately 10% to avoid generating too much heat and the formation of small metallic beads due to melting of alkali metal 103. When beads of alkali metal 103 float to the surface of the solution (*e.g.*, a solution including reactant 115), they continued to react with the solution, air, and water vapor, and in some cases may undesirably ignite the evolved hydrogen.

In some embodiments, the concentration of the PG/water solution may be adjusted (*e.g.*, "tuned") to obtain a desirable reaction rate. For example, in some embodiments, the concentration of the PG/water solution can be tuned such that the rate of formation of hydrogen is no faster than the rate at which the hydrogen egresses from the container. Hydrogen is a very small molecule and is therefore difficult to contain. Standard containers, such as a polyethylene bag with a slidable seal (*e.g.*, ZIPLOC®) or a sealable flap, may successfully contain used patch 101 and the solvent, and allow the hydrogen to pass through (or membranes, or Tyvek bags can be used as described above). However, when the reaction is allowed to proceed too quickly, hydrogen may form at a rate that exceeds the egress rate from the container. Accordingly, in some embodiments, a careful selection of the ratio of PG and water in solution may slow hydrogen production to a rate that it is matched by the egress rate from disposal container 105.

In some embodiments, reactant 115, or at least a portion of disposal kit 100 may include a color changing material that indicates when the reaction with alkali metal 103 is complete. More generally, reactant 115 or a portion of disposal kit 100 may include a material that changes physically in a perceptible way when the reaction is complete. The physical change could be triggered by heat, by pH of a solution or the reaction products contained therein, and the like. The color change could indicate to a user when the reaction is complete and when it is safe to dispose of disposal kit 100.

In some embodiments, disposal kit 100 may include an excess of a solution having a high thermal capacity or the container may include at least a portion of a wall made of a metal or other material with a high thermal capacity. In some embodiments, disposal kit 100 may include a label or a leaflet 120 (*e.g.*, stamped on the outside of one of the walls, or loosely placed inside the container, or attached through a string). Label or leaflet 120 may include a set of instructions for use. In some embodiments, the instructions may be directly printed on disposal container 105 or on a material that is disposed on an adhesive portion of used patch 101.

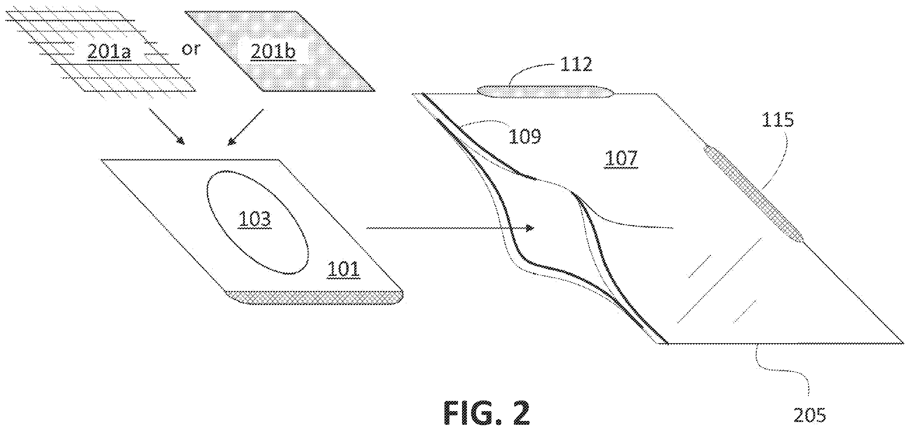

FIG. 2 illustrates a kit 200 for disposing of used patch 101 that is put in contact with a mesh 201a coated with reactant 115 (*e.g.*, stearyl alcohol), according to some embodiments. Mesh 201a may be a mesh, or a non-metallic mesh such as carbon fiber and the like. Mesh 201a offers a substrate for reactant 115, and also a good thermal conductivity that prevents over-heating of used patch 101 due to the neutralizing chemical reaction. In some embodiments, mesh 201a may be placed over used patch 101 and both then placed in cavity 107 inside a disposal pouch 205 (*cf.* disposal container 105). Disposal pouch 205 includes valve 112, reactant 115, and closeable opening 109. In some embodiments, mesh 201a may be replaced with a membrane 201b, paper, fiberglass, cloth layer, and the like, impregnated with a salt such as Epsom salt ($MgSO_4$ $7H_2O$), or other water-soluble salts such as $CdCl_2$ or similar. In some embodiments, mesh 201a or membrane 201b may include a permeable disposal means such as paper or a membrane to cover the adhesive surrounding the perimeter of alkali metal 103 on used patch 101. After neutralization of alkali metal 103, used patch 101 may be quenched with water and discarded.

In some embodiments, a suitably doped gel or wax is applied on used patch 101 prior to insertion in disposal pouch 205. Accordingly, the doped material may include a low concentration halogen (*e.g.*, Cl, F, Br, I, and the like) gradient in a gel or wax. The gradient may be formed upon exposure to room air. Alkali metal 103 would then react with the halogen in a controlled manner to slowly form a salt. Some embodiments may include a coat of an anhydrous foam over used patch 101; the foam physically restrains ejected sodium debris, which prevents combustion.

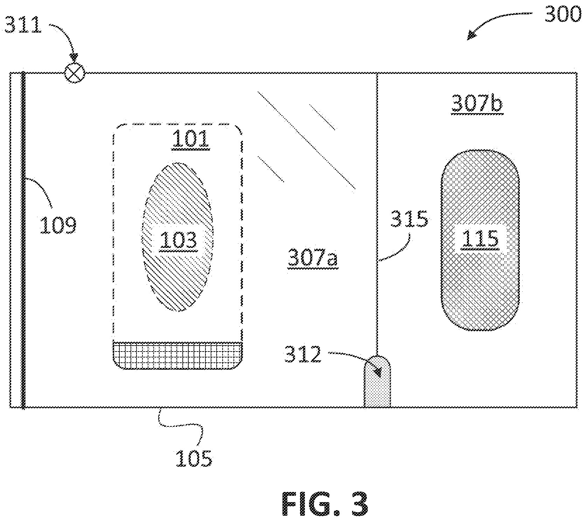

FIG. 3 illustrates a kit 300 for disposing of used patch 101 having alkali metal 103, including a first compartment or cavity 307a and a second compartment or cavity 307b (hereinafter, collectively referred to as "cavities 307"), according to some embodiments. Compartment 307b may include reactant 115 (or a solubilizer), separated from the used patch by a wall 315. A semi-permeable membrane 312 may cover at least a portion of wall 315. In some embodiments, separating reactant 115 from used patch 101 may reduce the reaction speed, which can safely proceed at a non-explosive rate.

In one embodiment, container 305 includes closeable opening 109. In one embodiment, container 305 may have a one-way valve 311, such as a duckbill valve, to allow a hydrogen egress from the container (or any other excess gas). One-way valve 311 is configured to open when subjected to a sufficient pressure differential between the inside of compartment 307a and the outside atmosphere. In some embodiments, one-way valve 311 is configured to open when pressure inside compartment 307a exceeds a threshold pressure. When the reaction product (*e.g.*, the alkali oxide or hydroxide product in the right hand side of Eq. 1) has the physical consistency of a solid or semi-solid form, one-way valve 311 may actuate at a lower pressure differential, or no pressure differential (*e.g.*, a flap valve). In some embodiments, one-way valve 311 may include a check valve or a pressure valve to allow for hydrogen egress from container 105.

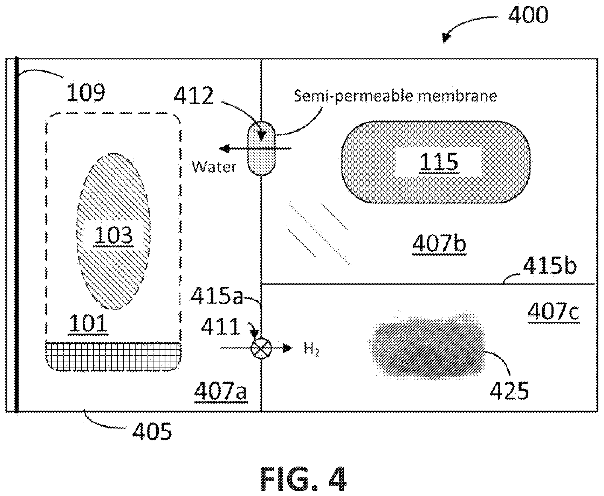

FIG. 4 illustrates a kit 400 for disposing of used patch 101 with alkali metal 103, including a first compartment 407a, a second compartment 407b, and a third compartment 407c (hereinafter, collectively referred to as "compartments 407"), according to some embodiments. In one embodiment, kit 400 includes a container 405 having closeable opening 109.

Third compartment 407c may include a hydrogen scavenger material 425, which reacts with free hydrogen to form a neutral compound. In some embodiments, scavenger material 425 may include a ceramic, a metal, or metal-oxide wire (*e.g.*, platinum-flashed alumina or similar), or any other material having a structure that traps the free hydrogen into a neutral configuration that may be disposed of via regular garbage disposal procedures. In some embodiments, scavenger material 425 may be a few mL of a solution such as dihydrolevoglucosenone (Cyrene), perfluorodecalin, cyclohexane, platinum, or palladium, among others.

Compartment 407b includes reactant 115 and is separated from compartment 407c by a wall 415b. A semi-permeable membrane 412 separates compartment 407b from compartment 407a, and allows water to flow from the latter to the former. In some embodiments, compartment 407c may be separated from compartment 407a via a hydrogen-permeable membrane 415a, or a one-way valve 411. In some embodiments, one-way valve 411 is activated when the difference in partial pressure of hydrogen between compartment 407a and compartment 407c exceeds a threshold value.

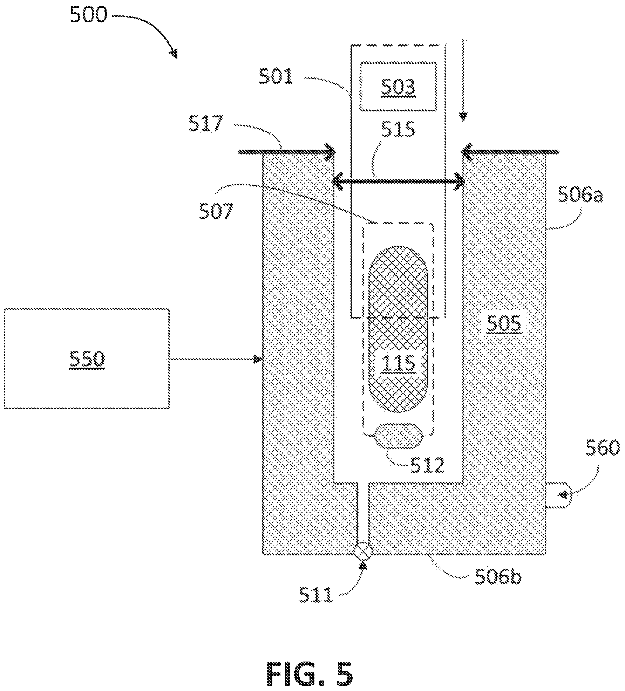

FIG. 5 illustrates a container 500 for disposing of used patch 501 including a cylindrical compartment 505, according to some embodiments. In some embodiments, cylindrical compartment 505 may include side walls 506a and a bottom wall 506b (hereinafter, collectively referred to as "outer walls 506") made of a hard plastic or other hard material (*e.g.*, a metal such as stainless steel and the like). In some embodiments, a metal tube forms a cylindrical compartment 505 with an inner annular space 515 of a porous body 507 filled with water or any other reactant 115. Accordingly, used patch 501 may be placed in annular space 515 so that reactant 115 slowly contacts alkali metal layer 503 on used patch 501 by porous body 507. Outer walls 506 may act as a thermal mass to constrain the overall temperature of the reaction. In some embodiments, the metal forming outer walls 506 is beneficial to limit the overall temperature increase of the system due to the high heat capacity of the metal. In some embodiments, the metal forms a thermal mass in the shape of a tube, and used patch 501 is placed inside, in the shape of a roll, with alkali metal 503 facing the interior of cylindrical compartment 505, where reactant 115 is located. More generally, some embodiments include containers 500 made from a material with a heat capacity and mass that effectively limits the rate of the reaction and the overall temperature increase inside container 500. In some embodiments, cylindrical compartment 505 may include a sintered metal, foil, or solid metal with or without holes or passages for water, or it could be a sufficient quantity of water or an aqueous solution or a non-aqueous solvent.

In some embodiments, container 500 may include a temperature control unit 550 to measure the temperature inside of the cylindrical compartment, or in the outer walls 506 of the compartment. The temperature measurement is an indicator of the energy released in the neutralization reaction (*cf.* Eq. 1) and may include a thermometer or a thermocouple. Accordingly, the temperature value may indicate whether the reaction is occurring too fast, or approaching an explosive or hazardous regime, and whether or not the reaction is complete.

In some embodiments, the opening of container 500 may be configured to close by a cap (*e.g.*, a threaded cap) or lid 517. In some embodiments, container 500 may further include a visual, audible, or electronic indicator or timer 560 that is activated when a sensor indicates that the neutralization reaction (*cf.* Eq. 1) is complete, or when it is safe to remove used patch 501 from container 500. In some embodiments, indicator 560 may respond to a temperature sensor, a pH detector, or a photodetector configured to detect the color of a portion of the used patch or the aqueous solution that has made contact with used patch 501. For example, an optical sensor may be configured to determine an ignition event. More specifically, an optical sensor may be configured to detect a light emission corresponding to at least one wavelength in the emission spectrum of alkali metal 503 (*e.g.*, approximately 589 nm for Na, approximately 794 nm for Rb, approximately 766 nm for K, and the like).

One-way valve 511 is configured to open when subjected to a sufficient pressure differential between inner annular space 515 and the outside atmosphere. A semi-permeable membrane 512 may cover at least a portion of porous body 507.

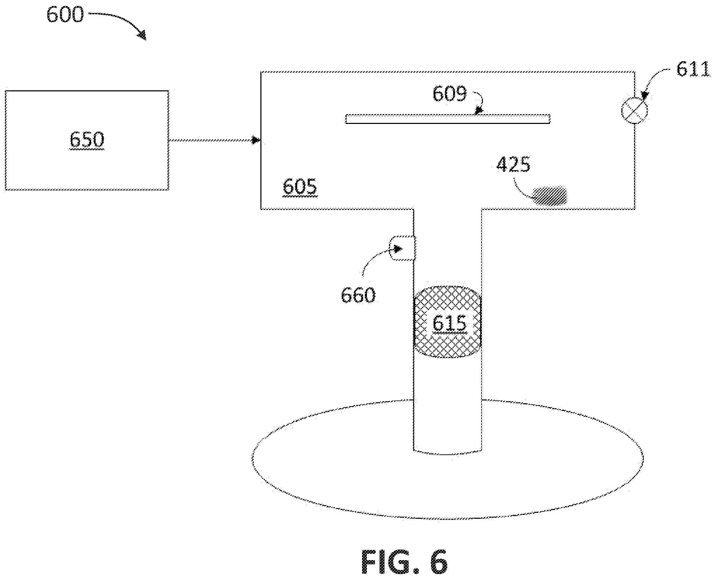

FIG. 6 illustrates a container 600 for disposing of multiple used patches, according to some embodiments. Container 600 includes an interior compartment 605 to receive the used patches through a one-way slot 609 configured as a closeable opening. In some embodiments, one-way slot 609 may include an automatic inlet driver (*e.g.*, a roller). In some embodiments, interior compartment 605 includes an atmosphere that is substantially filled with an inert gas (*e.g.*, argon or nitrogen) to mitigate any risk of an uncontrolled reaction due to atmospheric oxygen or water vapor. In some embodiments, up to a 10/90 air/argon atmosphere may be acceptable, even at temperatures higher than room temperature (*e.g.*, as high as 500 °F for Na). Further, in some embodiments, interior compartment 605 may be continually purged with a flow of an inert gas source 650 to reduce or eliminate the hydrogen resulting from the neutralization reaction of multiple patches. In addition, a one-way valve 611 may be included as described above, to allow the hydrogen gas to egress from compartment 605 while impeding or limiting the ingress of water vapor.

In some embodiments, container 600 may be semi-permanent, and receive multiple alkali patches. In some embodiments, container 600 may be wholly or partially replaced once a pre-selected number of alkali patches have been neutralized in its interior. In some embodiments, the interior of container 600 is at least partially filled with a reactant solution 615 (*e.g.*, in addition to the inert gas atmosphere) such as PG, and the like, as discussed above, or any other water evaporator. In some embodiments, the container is occasionally emptied of the used patches that have been neutralized (*e.g.*, 10-20 used patches). In some embodiments, the shape and size of container 600 (*e.g.*, base, neck, top, curvature, geometry, and the like), and the disposition of reactant solution 615 and closeable opening 609, may be configured to suppress any fire or ignition event in the interior. In that regard, some embodiments may include an alarm, indicator, or timer 660 with associated sensors, to indicate to a user that one or more, or all, of the used patches in the interior have been neutralized. Alarm or indicator 660 may also indicate that a temperature in the interior of container 600 has reached a hazardous level, or that an ignition has occurred or will occur imminently. In some embodiments, compartment 605 may include a scavenger material 425, as described above.

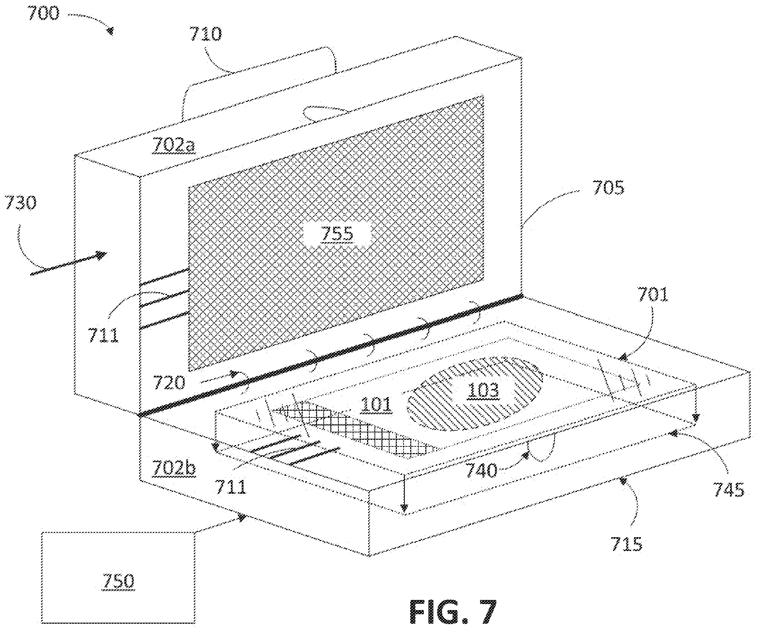

FIG. 7 illustrates a device 700 for disposing of a used patch 101, according to some embodiments. Device 700 includes a container 705 having a first platen 702a and a second platen 702b (hereinafter, collectively referred to as "platens 702"). One of platens 702 receives used patch 101 (*e.g.*, the bottom platen 702b). In some embodiments, used patch 101 could be loaded on platen 702b with a paper, or semi-permeable membrane 701 covering the adhesive and trapping alkali metal 103. In some embodiments, platens 702 are moveably connected. For example, in some embodiments, platens 702 are rotatably coupled through hinges 720 along one of the sides of either platen 702 using a handle 710. Platens 702 form a closed configuration, trapping used patch 101 in the plane between platens 702. In some embodiments, at least one of platens 702 includes a region manufactured from a material with a selected thermal conductivity and a selected thermal capacity. In some embodiments, at least one of platens 702 may include a material having a specific heat capacity of at least about the heat capacity of an alkali metal hydroxide at room temperature. Device 700 allows a rapid disposal of used patch 101 while absorbing a controlled amount of heat evolved, as platens 702 may be made of a high thermal capacity material.

In some embodiments, one of the platens (*e.g.*, the top platen) includes a substrate 755 having the reactant or solubilizer. In some embodiments, substrate 705 includes a polymer film, a non-woven material, a woven cloth, fiberglass, or paper. Accordingly, the reactant may include $MgSO_4$, $CdCl_2$, a water solution including any other salt, a water solution, alcohol, a glycol (*e.g.*, PG), or any other solvent as disclosed above. In some embodiments, container 705 may further include valves 711 (*e.g.*, a gas vent, one-way valve, or pressure valve), to release $H_2$ into the atmosphere. In some embodiments, the dissolution liquid is injected into the cavity via an injection point 730.

In some embodiments, a thermal mass 715 could also be placed on platen 702b in contact with the back side of used patch 101 (non-patient contacting side of the used patch) so that alkali metal 103 is not obstructed and can be completely reacted with the reactant on platen 702a. In some embodiments, device 700 also includes a temperature controller including a temperature sensor, as disclosed above. In some embodiments, temperature controller 750 may be coupled to an alarm or indicator that signals when the temperature has cooled sufficiently to be safe. The temperature controller may monitor the temperature that is passively limited from reaching a threshold value by selection and design of platens with sufficient thermal mass. In some embodiments, the temperature controller may actively control the flow of a coolant fluid to reduce the temperature in the platens, or use a thermo-electric cooling device.

In some embodiments, the device is configured to trap alkali metal 103 between two porous, non-flammable members with a high thermal capacity, to limit the overall temperature rise. Pure water can be introduced into this sandwich, which will react with alkali metal 103; however there will be no combustion of the hydrogen because the high heat capacity metal (with a high thermal conductivity) will limit the overall and local temperature increases. Some embodiments incorporate a lock 740 so that platens 702 lock together, the temperature and/or time is monitored, platens 702 unlock, and a visual or audible signal alerts the user to remove used patch 101.

In some embodiments, a rapid dissolution liquid (*e.g.*, water or water/PG mixture with 10% water or more) could be loaded into a sponge on platen 702a (or injected after the two platens are closed). Platens 702 could then be closed, trapping the reaction and allowing (with vents for the hydrogen) the thermal mass of the two platens to limit the reaction temperature maintaining a temperature below the melting point of alkali metal 103 and the auto-ignition temperature of the hydrogen. In some embodiments, platens 702 may form small vents when closed (*e.g.*, valves 711), to allow for hydrogen egress from the interior.

In some embodiments, the semi-permeable membrane covering the adhesive and also alkali metal 103 in used patch 101 has the benefit of trapping unreacted alkali metal to prevent beads of molten metal to egress from the device in case the reaction is sufficiently fast, such that the alkali metal melts. Some embodiments include a liquid disposal feature 745 such as a channel or trench so that any residual liquid (*e.g.*, alkali hydroxide and water) may be disposed of. Some embodiments may include a resident sponge to collect any residual liquid, to be disposed after use. Some embodiments may include a disposal container having a buffer solution to neutralize the alkali hydroxide that then may be emptied into standard waste streams or discarded as a unit.

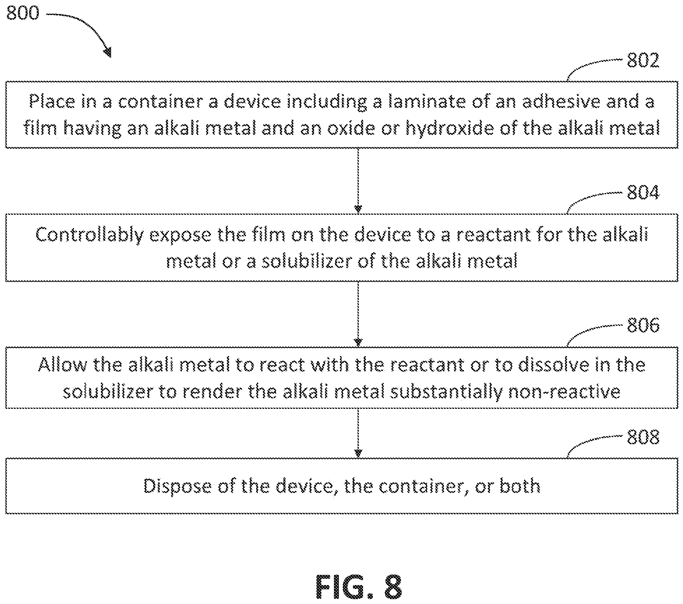

FIG. 8 is a flow chart illustrating steps in a method 800 for disposing of a used patch, according to some embodiments.

Step 802 includes placing into a container a device including a laminate of an adhesive and a film having an alkali metal layer or coating and an oxide or hydroxide of the alkali metal. In some embodiments, step 802 includes placing the device in a container through a closable opening in the container. In some embodiments, step 802 includes placing the device in a container including a wall and a bottom wall attached to the wall, wherein the wall includes a front wall and a rear wall, and the bottom wall is attached to the front wall and the rear wall.

Step 804 includes controllably exposing, or contacting, the film on the device to a reactant for the alkali metal or a solubilizer of the alkali metal.

In some embodiments, step 804 may include applying a low water concentration passivating gel with an applicator on the alkali metal. In some embodiments, step 804 includes supplying the low water concentration passivating gel in a packet that acts as its own applicator.

Step 806 includes allowing the alkali metal to react with the reactant or to dissolve in the solubilizer to render the alkali metal substantially non-reactive.

Step 808 includes optionally disposing of the device, the container, or both. In some embodiments, step 808 includes verifying a completion signal for the neutralization reaction from an indicator prior to disposing of the used device.

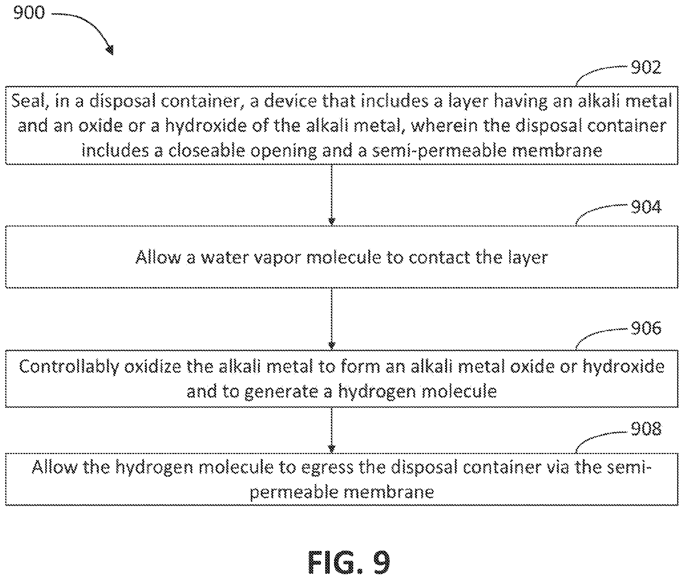

FIG. 9 is a flow chart illustrating steps in a method 900 for disposing of a used patch, according to some embodiments.

Step 902 includes sealing, in a disposal container, a device including a layer having an alkali metal and an oxide or a hydroxide of the alkali metal, wherein the disposal container includes a closeable opening and a semi-permeable membrane.

Step 904 includes allowing a water vapor molecule to contact the layer.

Step 906 includes controllably oxidizing the alkali metal to form an alkali metal oxide or hydroxide and to generate a hydrogen molecule.

Step 908 includes allowing the hydrogen molecule to egress the disposal container via the semi-permeable membrane.

As used herein, the phrase "at least one of" preceding a series of items, with the terms "and" or "or" to separate any of the items, modifies the list as a whole, rather than each member of the list (*e.g.*, each item). The phrase "at least one of" does not require selection of at least one item; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

To the extent that the term "include," "have," or the like is used in the description or the claims, such term is intended to be inclusive in a manner similar to the term "comprise" as "comprise" is interpreted when employed as a transitional word in a claim. The word "exemplary" is used herein to mean "serving as an example, instance, or illustration." Any embodiment described herein as "exemplary" is not necessarily to be construed as preferred or advantageous over other embodiments.

A reference to an element in the singular is not intended to mean "one and only one" unless specifically stated, but rather "one or more." All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public regardless of whether such disclosure is explicitly recited in the above description.

While this specification contains many specifics, these should not be construed as limitations on the scope of what may be claimed, but rather as descriptions of particular implementations of the subject matter. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

The subject matter of this specification has been described in terms of particular aspects, but other aspects can be implemented and are within the scope of the following claims. For example, while operations are depicted in the drawings in a particular order, this should not be understood as requiring that such operations be performed in the particular order shown or in sequential order, or that all illustrated operations be performed, to achieve desirable results. The actions recited in the claims can be performed in a different order and still achieve desirable results. As one example, the processes depicted in the accompanying figures do not necessarily require the particular order shown, or sequential order, to achieve desirable results. In certain circumstances, multitasking and parallel processing may be advantageous. Moreover, the separation of various system components in the aspects described above should not be understood as requiring such separation in all aspects, and it should be understood that the described program components and systems can generally be integrated together in a single software product or packaged into multiple software products. Other variations are within the scope of the following claims.

In one aspect, a method may be an operation, an instruction, or a function and vice versa. In one aspect, a claim may be amended to include some or all of the words (e.g., instructions, operations, functions, or components) recited in other one or more claims, one or more words, one or more sentences, one or more phrases, one or more paragraphs, and/or one or more claims.

The foregoing description is intended to illustrate various aspects of the instant technology. It is not intended that the examples presented herein limit the scope of the appended claims. The invention now being fully described, it will be apparent to one of ordinary skill in the art that many changes and modifications can be made thereto without departing from the spirit or scope of the appended claims.

To illustrate the interchangeability of hardware and software, items such as the various illustrative blocks, modules, components, methods, operations, instructions, and algorithms have been described generally in terms of their functionality. Whether such functionality is implemented as hardware, software, or a combination of hardware and software depends upon the particular application and design constraints imposed on the overall system. Skilled artisans may implement the described functionality in varying ways for each particular application.

As used herein, the phrase "at least one of" preceding a series of items, with the terms "and" or "or" to separate any of the items, modifies the list as a whole, rather than each member of the list (*e.g.*, each item). The phrase "at least one of" does not require selection of at least one item; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The word "exemplary" is used herein to mean "serving as an example, instance, or illustration." Any embodiment described herein as "exemplary" is not necessarily to be construed as preferred or advantageous over other embodiments. Phrases such as an aspect, the aspect, another aspect, some aspects, one or more aspects, an implementation, the implementation, another implementation, some implementations, one or more implementations, an embodiment, the embodiment, another embodiment, some embodiments, one or more embodiments, a configuration, the configuration, another configuration, some configurations, one or more configurations, the subject technology, the disclosure, the present disclosure, other variations thereof and alike are for convenience and do not imply that a disclosure relating to such phrase(s) is essential to the subject technology or that such disclosure applies to all configurations of the subject technology. A disclosure relating to such phrase(s) may apply to all configurations, or one or more configurations. A disclosure relating to such phrase(s) may provide one or more examples. A phrase such as an aspect or some aspects may refer to one or more aspects and vice versa, and this applies similarly to other foregoing phrases.

A reference to an element in the singular is not intended to mean "one and only one" unless specifically stated, but rather "one or more." Pronouns in the masculine (*e.g.*, his) include the feminine and neuter gender (*e.g.*, her and its) and vice versa. The term "some" refers to one or more. Underlined and/or italicized headings and subheadings are used for convenience only, do not limit the subject technology, and are not referred to in connection with the interpretation of the description of the subject technology. Relational terms such as first and second and the like may be used to distinguish one entity or action from another without necessarily requiring or implying any actual such relationship or order between such entities or actions. All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public regardless of whether such disclosure is explicitly recited in the above description. No claim element is to be construed under the provisions of 35 U.S.C. §112, sixth paragraph, unless the element is expressly recited using the phrase "means for" or, in the case of a method claim, the element is recited using the phrase "step for."

While this specification contains many specifics, these should not be construed as limitations on the scope of what may be described, but rather as descriptions of particular implementations of the subject matter. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially described as such, one or more features from a described combination can in some cases be excised from the combination, and the described combination may be directed to a subcombination or variation of a subcombination.

The title, background, brief description of the drawings, abstract, and drawings are hereby incorporated into the disclosure and are provided as illustrative examples of the disclosure, not as restrictive descriptions. It is submitted with the understanding that they will not be used to limit the scope or meaning of the claims. In addition, in the detailed description, it can be seen that the description provides illustrative examples and the various features are grouped together in various implementations for the purpose of streamlining the disclosure. The method of disclosure is not to be interpreted as reflecting an intention that the described subject matter requires more features than are expressly recited in each claim. Rather, as the claims reflect, inventive subject matter lies in less than all features of a single disclosed configuration or operation. The claims are hereby incorporated into the detailed description, with each claim standing on its own as a separately described subject matter.

The claims are not intended to be limited to the aspects described herein, but are to be accorded the full scope consistent with the language claims and to encompass all legal equivalents. Notwithstanding, none of the claims are intended to embrace subject matter that fails to satisfy the requirements of the applicable patent law, nor should they be interpreted in such a way.

## LIST OF EMBODIMENTS

Embodiment 1. A method for disposing of a device comprising an alkali metal, comprising:

> placing into a container a device comprising a layer portion having at least an alkali metal, an oxide of the alkali metal, a hydroxide of the alkali metal, or any combination thereof;
> controllably exposing the layer portion on the device to a reactant for the alkali metal or a solubilizer of the alkali metal;
> allowing the alkali metal to react with the reactant or to dissolve in the solubilizer to render the alkali metal substantially non-reactive; and
> optionally disposing of the device, the container, or both.

Embodiment 2. The method of Embodiment 1, wherein the device is a medical device and placing in a container the device comprises first applying the medical device to a portion of a skin tissue in a patient.

Embodiment 3. The method of Embodiment 1 or Embodiment 2, wherein said placing comprises placing the device in a container through a closable opening in the container.

Embodiment 4. The method of Embodiment 1 or Embodiment 2, wherein the said placing comprises placing the device in a container comprising a wall and a bottom wall attached to the wall.

Embodiment 5. The method of Embodiment 1 or Embodiment 2, wherein said placing comprises placing the device in a container comprising a first platen and a second platen, one of said platens configured to receive the device, the method further comprising rotatably compressing the first platen against the second platen.

Embodiment 6. The method any one of Embodiments 1-5, further comprising controlling the temperature of the container during said allowing.

Embodiment 7. The method of any one of Embodiments 1-6, further comprising disposing of the container after a single-use.

Embodiment 8. The method of any preceding Embodiment, further comprising placing a second device in the container.

Embodiment 9. The method of any preceding Embodiment, wherein controllably exposing the layer portion on the device to a reactant comprises controllably oxidizing the alkali metal to form an alkali metal oxide or hydroxide and to generate a hydrogen molecule.

Embodiment 10. The method of any preceding Embodiment, wherein allowing the alkali metal to react with the reactant comprises allowing a hydrogen molecule to egress the disposal container.

Embodiment 11. A kit, comprising:

> a device comprising a layer of an alkali metal; and
> a disposal container comprising a cavity configured to receive the device and an opening configured to receive the device into the cavity, the opening closable or capable of being closed, wherein the disposal container comprises one or more of:

> > (i) a solvent that dissolves the alkali metal;
> > (ii) a means for the egress of hydrogen or a scavenger of hydrogen; and
> > (iii) a source of reactant or a mechanism to receive water from an external source of water.

Embodiment 12. The kit of Embodiment 11, wherein the device is removably within a packaging container that is impermeable to water and to air, and wherein the packaging container is comprised of a metal foil, the packaging container comprises an interior compartment to receive the device, and the interior compartment has an atmosphere that is substantially argon or nitrogen, and the disposable container comprises an exit for egress of hydrogen in the form of a hydrogen permeable polymer membrane.

Embodiment 13. The kit of Embodiment 12, wherein the polymer membrane is also permeable to water vapor, and the polymer membrane is comprised of polytetrafluoroethylene.

Embodiment 14. The kit of Embodiment 11, further comprising a solvent that is miscible with water and that dissolves the alkali metal, wherein the solvent is an alcohol or a glycol, and wherein the alcohol is selected from ethanol, isopropanol, t-butanol, stearyl alcohol, and tris(trimethylsilyl)methanol, wherein the glycol is propylene glycol.

Embodiment 15. The kit of Embodiment 11, wherein the kit comprises a source of water, wherein the source of water is water or a water solution in the disposal container, or wherein the water or water solution is contained on or in a sponge, a porous body, or an absorbent polymer substrate, or wherein the porous body is comprised of a sintered metal or a porous polymer, or wherein the absorbent polymer substrate is a hydrogel comprised of a cross-linked hydrophilic polymer.

Embodiment 16. The kit of Embodiment 11, wherein the kit comprises a mechanism to receive water from a source of water external to the disposal container, wherein the mechanism to receive water from an external source is a water or moisture permeable polymer membrane.

Embodiment 17. The kit of Embodiment 11, further comprising a container with an aqueous solution, a triglyceride, an anhydrous foam, or a compound with a counter ion that produces upon reacting with sodium a compound selected from sodium alginate, sodium bifluoride, sodium fluorosilicate, sodium metaborate, sodium paraperiodate, sodium stearate, sodium zirconium glycolate, and sodium perrhenate.

Embodiment 18. The kit of Embodiment 11, wherein the device further comprises an adhesive layer or perimeter, and the kit further comprises a substrate dimensioned for receiving the adhesive layer or perimeter.

Embodiment 19. A container, comprising:

an enclosure forming a cavity and configured to receive a device comprising an alkali metal layer in the cavity;
a medium configured to allow contact of a water molecule or a reactant with the alkali metal at a pre-selected rate or in a controlled manner;
a semi-permeable membrane that prevents water from exiting the container, and a one-way valve responsive to pressure to allow hydrogen to escape the container; and
a water-vapor generator configured to provide the water molecule.

Embodiment 20. The container of Embodiment 19, wherein the medium includes at least one of:
a membrane having a diffusion gradient selected according to the pre-selected rate,

a membrane configured to trap a molten bead of the alkali metal and prevent it from detaching from the mass of alkali metal or from floating to a surface of the medium, a water solution forming a gel, and
a sponge, a bead, a gel or a substrate that traps the water molecule, and a porous sintered metal.

## Claims

1. A method for deactivating a device comprising an alkali metal, comprising:

placing into a container a device comprising an alkali metal, wherein the container comprises a cavity and a closeable opening, the cavity configured to receive the device via the opening;
contacting the device to a reactant or solubilizer for neutralizing the alkali metal, and
disposing of the device, the container, or both upon rendering the alkali metal substantially non-reactive.

2. The method of claim 1, wherein the device is a disposable patch.

3. The method of claim 1 or claim 2, wherein the contacting converts the alkali metal to a non-pyrophoric substance comprising one or more of a metal hydroxide, a metal alkoxide, and a salt.

4. The method of any one of claims 1-3, wherein the reactant or solubilizer comprises a solvent that dissolves the alkali metal, optionally wherein the solvent comprises an alcohol or a glycol.

5. The method of claim 4, wherein the reactant or solubilizer comprises propylene glycol, optionally wherein the reactant or solubilizer further comprises water.

6. The method of claim 4 or claim 5, wherein the reactant or solubilizer further comprises a buffer or an acid.

7. The method of any one of claims 1-6, wherein the container comprises the reactant or solubilizer.

8. The method of any one of claims 1-7, wherein the contacting comprises partially or totally immersing the device in the reactant or solubilizer.

9. The method of any one of claims 1-8, further comprising placing a membrane or paper over the device prior to placing the device into the container.

10. The method of any one of claims 1-9, further comprising placing a second device comprising a second alkali metal into the container.

11. A disposal container, comprising:

   a cavity and a closeable opening, the cavity configured to receive a device comprising an alkali metal via the opening;
   a reactant or solubilizer for neutralizing the alkali metal; and
   an exit for egress of hydrogen for release into the atmosphere.

12. The disposal container of claim 11, wherein the opening is configured to close by a cap or a lid.

13. The disposal container of claim 11 or claim 12, wherein the cavity configured to receive the device is a circular or an annular cavity.

14. The disposal container of any one of claims 11-13, wherein the exit is selected from a valve, a vent, the closeable opening, and a hydrogen permeable polymer membrane, optionally wherein the valve is responsive to pressure.

15. A kit, comprising:

   a device comprising an alkali metal; and
   the disposal container of any one of claims 11-14.

**FIG. 1**

FIG. 2

EP 4 403 205 A2

**FIG. 3**

EP 4 403 205 A2

FIG. 4

EP 4 403 205 A2

FIG. 5

FIG. 6

EP 4 403 205 A2

FIG. 7

800 —

802

Place in a container a device including a laminate of an adhesive and a film having an alkali metal and an oxide or hydroxide of the alkali metal

804

Controllably expose the film on the device to a reactant for the alkali metal or a solubilizer of the alkali metal

806

Allow the alkali metal to react with the reactant or to dissolve in the solubilizer to render the alkali metal substantially non-reactive

808

Dispose of the device, the container, or both

# FIG. 8

900 —

902

Seal, in a disposal container, a device that includes a layer having an alkali metal and an oxide or a hydroxide of the alkali metal, wherein the disposal container includes a closeable opening and a semi-permeable membrane

904

Allow a water vapor molecule to contact the layer

906

Controllably oxidize the alkali metal to form an alkali metal oxide or hydroxide and to generate a hydrogen molecule

908

Allow the hydrogen molecule to egress the disposal container via the semi-permeable membrane

## FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62946124

**Non-patent literature cited in the description**

- **PULYALINA A. et al.** *Polymers,* 2018, vol. 10 (8), 828